# FASCICULE DE BREVET EUROPEEN

(11) **EP 3 013 791 B1**
(45) Date de publication et mention de la délivrance du brevet: **17.01.2018**
(21) Numéro de dépôt: 14735630.7
(22) Date de dépôt: 16.06.2014
(51) Int. Cl.: C07C 233/47, C07C 237/22, C11D 1/52, B01J 13/00, C09D 5/00, C09D 7/00

(54) **AMIDE GRAS A BASE DE CAPROLACTAME COMME ADDITIF HYDROGELATEUR.**
CAPROLACTAMBASIERTES FETTSÄUREAMID ALS HYDROGELIERUNGSADDITIV
CAPROLACTAM-BASED FATTY AMIDE AS HYDROGELLING ADDITIVE

(30) Priorité: 28.06.2013 FR 1356315
(43) Date de publication de la demande: 04.05.2016
(73) Titulaire: Arkema France, 92700 Colombes (FR)
(72) Inventeur: BERNARD, Michael Y., F-95880 Enghien-les-bains (FR)
(74) Mandataire: Killis, Andréas
(86) Numéro de dépôt international: PCT/FR2014/051471
(87) Numéro de publication internationale: WO 2014/207343

(56) Documents cités:
- M. SUZUKI, ET AL.: "L-Lysine-based low-molecular-weight gelators", CHEMICAL SOCIETY REVIEWS, vol. 38, no. 4, 9 février 2009 (2009-02-09), pages 967-975, XP055116388, Royal Society of Chemistry, Cambridge, GB ISSN: 0306-0012, DOI: 10.1039/b816192e cité dans la demande
- A.I. RAKHIMOV, ET AL.: "Importance of acyl rearrangement in the acid-catalysed reaction of [epsilon]-caprolactam with carboxylic acids", RUSSIAN JOURNAL OF ORGANIC CHEMISTRY, vol. 42, no. 8, août 2006 (2006-08), pages 1252-1253, XP019407576, Nauka/Interperiodica ISSN: 1608-3393, DOI: 10.1134/s1070428006080318

## Description

La présente invention concerne un additif amide gras porteur d'une terminaison acide carboxylique ou sous forme de sel et utilisé en tant qu'hydrogélateur pour compositions aqueuses, en particulier pour revêtements ou colles ou adhésifs ou traitement de fibres ou de textiles ou pour détergents, décapants, dépolluants, floculants.

Divers systèmes pouvant gélifier dans l'eau sont déjà connus et utilisés dans diverses applications.

FR 2976948 décrit une association ternaire d'un acide de bore, comme l'acide borique, avec un N-alkyl aldonamide, en particulier N-dodécyl-D-glucoanamide et d'un sel monovalent tel que NaCl pour l'obtention de gels en milieu aqueux (salin) pour des applications diverses et en particulier dans la détergence.

Parmi les hydrogélateurs connus, figurent ceux à base de dérivés de lysine comme décrits par M.Suzuki et al dans Chem. Soc. Rev., 2009, 38, 967-975, où sont décrits également des dérivés utilisés comme organogélateurs. Parmi ces dérivés sont décrits des diurées-esters, diamide esters ou amide-urée-esters à partir de lysine.

Une revue d'hydrogélateurs a été réalisée par L.A.Estroff et al dans Chemical Reviews, 2004, 104, 3, 1201-1217 avec un inventaire des méthodes de caractérisation et des structures connues. Sont distingués entre autres les dérivés amphiphiles conventionnels ayant une tête hydrophile et une ou deux chaînes hydrophobes, les bolaamphiphiles ayant deux têtes hydrophiles liées par une chaîne hydrophobe, les tensioactifs à double tête ionique séparée par un espaceur rigide avec deux chaînes terminales flexibles, systèmes dérivés des sucres. Il est reconnu qu'il n'y a pas de règle générale applicable qui permet de trouver le bon compromis entre hydrophilie et hydrophobie d'une molécule et en conséquence il n'y a pas non plus de règle générale entre l'aptitude à former un gel en milieu aqueux et la tendance à éviter la précipitation des fibres.

L'inconvénient de ces hydrogélateurs est le fait qu'ils sont à base d'aminoacides qui peuvent subir des réactions secondaires et en particulier des allongements de chaîne incontrôlés suivant les conditions de préparation desdits gélateurs et ainsi affecter leur structure fine et par conséquent leur performance de gélateur. D'autre part, aucun des documents cités ne décrit ni n'enseigne comment obtenir des additifs amides modifiés par le caprolactame et avec des performances améliorées objet de la présente invention pour remédier aux inconvénients de l'état de la technique.

En effet, la présente invention cherche à mettre au point des nouveaux additifs amides modifiés par une structure caprolactame (équivalente à un amino acide en C₆) sans avoir recours à des acides aminés condensés sur eux-mêmes ou à des acides et des amines réagissant par polycondensation avec des sous-produits à éliminer. Ces amides modifiés permettent l'utilisation d'un procédé de préparation simple et pratique à mettre en oeuvre, avec l'ouverture contrôlée du cycle du caprolactame, afin d'éviter des réactions secondaires difficiles à éviter avec un aminoacide équivalent et sans besoin d'étapes de séparation et/ou de purification du produit final, lequel présente des performances rhéologiques satisfaisantes en milieu aqueux, sans affecter les performances propres des liants aqueux auxquels il peut être associé.

Le premier objet de l'invention concerne un additif hydrogélateur à base d'amide gras, lequel additif est le produit de réaction d'addition (sans sous produits comme en polycondensation) d'un acide gras R₁CO₂H, sur du caprolactame et (lequel produit) porte une fonction carboxylique terminale sous forme d'acide ou sous forme salifiée par un agent neutralisant, ledit additif étant constitué de ou comprenant le mélange de 3 composés amides différents, résultant de ladite réaction et caractérisés par le nombre n d'unités caprolactame incorporées, lequel nombre est respectivement de 1, de 2 et de 3, avec un nombre n moyen d'unités (moyenne par molécule) allant de 0,8 à 3, de préférence de 0,9 à 2,75 et plus préférentiellement allant de 0,9 à 2,5. Plus particulièrement, lesdits 3 composés peuvent être définis selon la formule (I) suivante et correspondent respectivement à n = 1, n = 2 et n = 3

R₁-C(=O)-[NH(CH₂)₅C(=O)-]ₙ₋₁-NH(CH₂)₅-CO₂M (I)

avec M étant -H ou un cation dans le cas de ladite forme salifiée, ledit cation étant lié audit agent neutralisant.

Ledit acide gras R₁CO₂H comprend un nombre d'atomes de carbone allant de 10 à 24, ce qui signifie que R₁ est un alkyl comprenant de 9 à 23 atomes de carbone, de préférence linéaire.

Comme exemples convenables de R₁CO₂H, on peut citer les monoacides gras tels que l'acide décanoïque à l'acide undécanoïque, l'acide dodécanoïque ou laurique, l'acide tridécanoïque, l'acide tetradécanoïque (ou myristique), l'acide pentadécanoïque, l'acide hexadécanoïque (acide palmitique), l'acide heptadécanoïque, l'acide octadécanoïque (acide stéarique) ou leurs isomères et leurs mélanges.

L'acide gras R₁CO₂H peut également comprendre dans sa structure une insaturation éthylénique, comme c'est le cas de l'acide oléique (acide 9-octadécène-oïque).

R₁CO₂H peut comprendre dans sa structure une fonction hydroxyle, c'est-à-dire être un hydroxyacide gras, comme l'acide 12-hydroxy stéarique, 9-hydroxy stéarique et/ou l'acide 10-hydroxy stéarique ou l'acide 14-hydroxy eicosanoïque, de préférence l'acide 12-hydroxy stéarique seul ou remplacé en partie par l'acide 9-hydroxy stéarique et/ou l'acide 10-hydroxy stéarique ou l'acide 14-hydroxy eicosanoïque. R₁CO₂H peut comprendre à la fois une insaturation éthylénique et une fonction OH, comme par exemple l'acide ricinoléique (ou acide 12-hydroxy 9-octadécène-oïque) ou l'acide 14-hydroxy eicosénoïque. De préférence, ledit acide gras R₁CO₂H ne comprend aucune fonction hydroxyle (OH) ou amine ou autre fonction pouvant réagir avec le caprolactame afin d'éviter toute réaction potentielle secondaire et mieux contrôler la structure et composition finale dudit additif amide selon l'invention.

Selon une option possible, ledit additif amide est sous forme salifiée (de sel d'acide carboxylique) et ledit agent neutralisant est sélectionné parmi les bases organiques ou minérales. Comme exemples de bases organiques, on peut citer des aminés, primaires ou secondaires ou tertiaires, de préférence amines tertiaires, pour former un cation M qui est un ammonium quaternaire, le contre anion étant le carboxylate porté par la chaîne amide. Comme base minérale, on peut citer un hydroxyde de métal alcalin comme LiOH, NaOH, KOH ou alcalinoterreux comme Ca(OH)₂, Mg(OH)₂, Ba(OH)₂.

Plus particulièrement, l'additif de l'invention peut être sous la forme d'une poudre micronisée, de préférence ayant une taille moyenne en volume inférieure à 50 µ, de préférence inférieure à 25 µ. Cette granulométrie peut être déterminée directement sur la poudre sèche par diffraction laser comme par exemple sur le Mastersizer® S de Malvern. Cette technique est basée sur le principe que des particules passant à travers un faisceau laser diffractent la lumière selon un angle différent en fonction de leur taille : les particules de petites tailles diffractent aux grands angles, alors que les particules de tailles plus importantes diffractent aux petits angles.

Ledit additif de l'invention peut être utilisé en particulier sous forme de gel dans l'eau ou en tant qu'hydrogélateur et plus préférentiellement à un taux en poids inférieur à 5% et encore plus préférentiellement à un taux ne dépassant pas 1% en poids, ce pourcentage étant défini par rapport au poids de l'eau + additif.

Le deuxième objet de l'invention concerne un procédé de préparation de l'additif hydrogélateur selon l'invention, lequel procédé comprend une étape de réaction d'addition entre un acide gras R₁CO₂H et le caprolactame avec un rapport molaire dudit caprolactame par rapport audit acide gras allant de 0,8 à 3, de préférence de 0,9 à 2,75 et plus préférentiellement de 0,9 à 2,5, ledit procédé ne comprenant aucune étape de séparation ni de purification.

Cette réaction est une réaction d'addition en masse à l'état fondu qui peut avoir lieu à une température allant de 200°C à 300°C et sous atmosphère inerte ou sous pression allant de quelques bars à une pression inférieure à 30 bars. Un catalyseur peut être utilisé pour cette réaction comme par exemple un acide de Lewis. Après refroidissement, le produit obtenu est micronisé par broyage mécanique ou par jet d'air à une température inférieure à la température de fusion. Un tamisage peut permettre d'obtenir une granulométrie fine et contrôlée avec une taille moyenne en volume inférieure à 50 µ, de préférence inférieure à 25 µ mesurée par diffraction laser comme par exemple sur le Mastersizer® S de Malvern®.

Un autre objet de l'invention concerne une composition aqueuse qui comprend en tant qu'hydrogélateur, au moins un additif tel que défini ci-haut ou tel qu'obtenu par le procédé décrit de l'invention.

Une telle composition aqueuse peut être une composition aqueuse avec ou sans liant. Sans liant, cela peut correspondre à une simple dispersion dudit additif dans l'eau lequel fonctionne comme hydrogélateur. L'effet hydrogélateur se manifeste par une augmentation de viscosité avec obtention d'un gel, par le fait des associations moléculaires dans le système fibreux formé, avec l'hydrogélation étant un compromis entre hydrophobie/hydrophilie moléculaire de l'additif d'une part et d'autre part entre le niveau de solubilisation (limité mais suffisant) et une tendance à la précipitation à éviter. Cela peut aussi correspondre à une solution ou dispersion de dispersant ou tensioactif aqueux pour les besoins de détergence ou de dispersion d'un produit huileux (huiles d'origine végétale ou minérale ou hydrocarbures) ou solide finement divisé (dispersion de résines sous forme particulaire).

Ladite composition aqueuse peut être une composition de liant organique qui peut être réactif ou non réactif, monocomposant ou bicomposant, monomère ou oligomère ou résine ou polymère ou mélange de monomères et d'oligomères ou résines ou polymères. Comme il s'agit de milieu aqueux, lesdits liants peuvent être hydrosolubles, hydrodispersibles par leur propre structure ou dispersibles dans l'eau à l'aide d'un tensioactif ou dispersant spécifique pour milieu aqueux.

En particulier, le pH de la composition aqueuse est basique, de préférence d'au moins 9, plus préférentiellement d'au moins 10. Ce pH est réglé par addition en excès d'une base, c'est-à-dire au delà de la simple neutralisation de ladite fonction terminale acide carboxylique dudit additif et ceci reste également valable pour l'additif sous forme de gel dans l'eau.

Selon une première option préférée, ladite composition est une composition aqueuse de liant et de préférence une composition aqueuse de revêtements, en particulier de vernis, de peintures, d'encres ou une composition de colles ou d'adhésifs ou de cosmétique ou une composition de traitement de fibres ou de textiles, avec ledit additif hydrogélateur étant utilisé comme additif de rhéologie.

Selon une autre option, ladite composition aqueuse est une composition de tensioactif en particulier de détergent ou d'agent décapant ou d'agent dépolluant ou d'agent floculant. Comme dans les compositions aqueuses de liant, ledit additif amide est présent en tant qu'hydrogélateur pour gélifier la composition d'application pour des raisons propres à l'utilisation finale projetée de la composition applicative.

Un autre objet de l'invention concerne l'utilisation d'un additif tel que défini ci-haut selon l'invention en tant qu'additif hydrogélateur dans des compositions aqueuses. Une telle utilisation est déjà décrite ci-dessus, en particulier dans des compositions aqueuses de liant, plus particulièrement dans des compositions aqueuses de revêtements ou de colles ou d'adhésifs ou de cosmétique ou de composition de traitement de fibres ou de textiles, en particulier comme additif de rhéologie. Une autre utilisation dans des compositions aqueuses concerne les compositions de tensioactif et en particulier de détergent, d'agent décapant ou d'agent dépolluant ou d'agent floculant. Finalement sont également couverts par l'invention les produits finis résultant de l'utilisation comme hydrogélateur dudit additif de l'invention, en particulier sélectionné parmi : un revêtement ou une colle ou un adhésif ou un cosmétique ou une fibre traitée ou un textile traité ou un détergent ou un agent décapant ou un agent dépolluant ou un agent floculant. L'hydrogel en tant que produit qui résulte de ces utilisations est également couvert par l'invention.

Les exemples qui suivent sont présentés à titre d'illustration de l'invention et de ses performances et ne limitent en rien sa couverture.

### Partie expérimentale

### I - Matières premières utilisées

**Tableau 1 : Matières premières utilisées**

| **Produit** | **Fonction** | **Référence commerciale** | **Fournisseur** |
|---|---|---|---|
| Acide Stéarique | Réactif | Stearic acid | ALDRICH |
| Caprolactame | Réactif | ε-Caprolactam | ALDRICH |
| Solution de soude 1.0 M | Acide | Sodium hydroxide solution ∼1.0 M | ALDRICH |

### II - Exemples de préparation des hvdrogélateurs à base de caprolactame

### Exemple 1 : préparation de l'amide A1 par réaction de 1 mole d'acide stéarique avec 1 mole de Caprolactame :

Dans un réacteur de 1-Litre équipé d'un thermomètre, d'un condensateur et d'un agitateur, on introduit sous un courant d'azote, 113,16 g de Caprolactame (1 mole), 284,48 g d'acide stéarique (1 mole). Le mélange est ensuite chauffé à 250°C, toujours sous courant d'azote. La réaction est contrôlée par la viscosité. Après 15 heures, la valeur de viscosité devient constante (> 0,32 Poises ou > 32 mPa.s, mesuré sur un Brookfield® CAP1000 à 120°C), le mélange réactionnel est refroidi à 150°C puis déchargé dans un moule siliconé. Une fois refroidi à la température ambiante, le produit est micronisé mécaniquement par broyage. Un tamisage pour obtenir une granulométrie fine et contrôlée avec taille moyenne obtenue de 7 µm est effectué.

### Exemple 2 : préparation de l'amide A2 par réaction de 1 mole d'acide stéarique sur 2 moles de Caprolactame :

Dans un réacteur de 1-Litre équipé d'un thermomètre, un condensateur et d'un agitateur, on introduit sous un courant d'azote, 226,32 grammes de Caprolactame (soit 2 moles), 284,48 grammes d'acide stéarique (soit 1 mole).

Le reste du mode opératoire est identique à celui décrit pour l'exemple 1.

### III - Evaluation des performances d'hydrogélateur

### 1. Formulation pour l'évaluation des hydrogélateurs

Dans un erlenmeyer muni d'un barreau aimanté, on introduit 99 grammes d'eau déminéralisée, 1 gramme d'additif tel que préparé selon exemples 1 et 2 (hydrogélateur) broyé ou micronisé à tester, puis quelques gouttes de soude concentrée en quantité supérieure à 4 fois en équivalent l'acide carboxylique de l'hydrogélateur à tester. L'erlenmeyer est ensuite fermé. Puis, le mélange est agité pendant plus de 5 heures à 85°C pour avoir une parfaite dissolution de l'additif hydrogélateur et un mélange laiteux mais sans précipité. Enfin, le mélange est introduit dans un tube à essai puis laissé au repos à 25°C pendant 24 heures.

Dans ces conditions de préparation, 3 formulations ont été réalisées avec l'amide A1 de l'exemple 1, l'amide A2 de l'exemple 2 et aussi avec l'acide stéarique à titre de référence de comparaison. Ces 3 essais sont résumés dans le tableau 2 qui suit.

**Tableau 2 : Formulations**

| **Réf. Essai** | **Additif hydrogélateur ou de référence (comparatif)** | **% en poids d'additif vs formulation** |
|---|---|---|
| 1 | Acide stéarique (référence comparative) | 1% |
| 2 | A1 (exemple 1 selon l'invention) | 1% |
| 3 | A2 (exemple 2 selon l'invention) | 1% |

### 2. Evaluation du gel

Les 3 essais de formulations ont été évalués de deux manières : d'abord selon l'aspect des formulations préparées dans les tubes à essais après 24 heures (voir tableau 3) et ensuite et surtout selon leur viscosité à différentes vitesses de cisaillements sur un viscosimètre Brookfield® (voir tableau 4).

**Tableau 3 : Aspect**

| **Réf Essais** | **Aspect** |
|---|---|
| 1 | Suspension |
| 2 | Gel |
| 3 | Gel |

**Tableau 4 : Viscosité à différentes vitesses**

| | Vitesse mobile (rpm) | Essai 1 (comparatif) | Essai 2 (invention) | Essai 3 (Invention) |
|---|---|---|---|---|
| Indice Thixo TI 1/10 | | 6,03 | 7,01 | 5,23 |
| Indice Thixo TI 5/50 | | 5,17 | 7,06 | 6,16 |
| Viscosité mesurée | 1 | 3200* | 6100 | 3400 |
| | 5 | 900* | 1880 | 1510 |
| Brookfield® à 25°C (mPa.s) | 10 | 530* | 870 | 650 |
| | 50 | 174* | 266 | 245 |
| | 100 | 116 | 170 | 126 |

| | | | | |
|---|---|---|---|---|
| * A titre indicatif, il faut noter que les résultats indiqués pour l'essai 1 comparatif ne sont pas très reproductibles étant donné que la formulation est non homogène. | | | | |

Contrairement à l'acide stéarique, les formulations obtenues avec 1% d'amide A1 ou A2 se présentent sous forme de gel caractéristique d'un hydrogélateur.

Quant aux résultats de viscosité, ils montrent bien que la formulation contenant l'amide A1 (essai 2) est thixotropique mais également plus visqueuse que l'eau ou la formulation contenant l'acide stéarique.

## Revendications

1. Additif hydrogélateur à base d'amide gras, **caractérisé en ce qu'**il est le produit de réaction d'addition d'un acide gras R₁CO₂H sur du caprolactame et porte une fonction carboxylique terminale sous forme acide ou sous forme salifiée par un agent neutralisant et que ledit additif est constitué de ou comprend le mélange de 3 composés amides différents résultant de ladite réaction et **caractérisés par** le nombre n d'unités caprolactame incorporées, lequel nombre est respectivement de 1, de 2 et de 3, avec un nombre n moyen d'unités (moyenne par molécule) allant de 0,8 à 3, de préférence de 0,9 à 2,75 et plus préférentiellement allant de 0,9 à 2,5.

2. Additif selon la revendication 1, **caractérisé en ce que** lesdits 3 composés sont définis selon la formule (I) suivante et correspondent respectivement à n = 1, n = 2 et n = 3
R₁-C(=O)-[NH(CH₂)₅C(=O)-]ₙ₋₁-NH(CH₂)₅-CO₂M (I)
avec M étant -H ou un cation dans le cas de ladite forme salifiée, ledit cation étant lié audit agent neutralisant.

3. Additif selon la revendication 1 ou 2, **caractérisé en ce que** ledit acide gras comprend un nombre d'atomes de carbone allant de 10 à 24.

4. Additif selon l'une des revendications 1 à 3, **caractérisé en ce que** ledit additif est sous la forme d'une poudre micronisée, de préférence ayant une taille moyenne en volume inférieure à 50 µ , de préférence inférieure à 25 µ.

5. Additif selon l'une des revendications 1 à 4, **caractérisé en ce qu'**il est sous forme salifiée et que ledit agent neutralisant est sélectionné parmi les bases organiques ou minérales.

6. Additif selon l'une des revendications 1 à 5, **caractérisé en ce qu'**il est sous forme de gel dans l'eau ou en tant qu'hydrogélateur et plus préférentiellement à un taux en poids inférieur à 5% et encore plus préférentiellement à un taux ne dépassant pas 1% en poids.

7. Procédé de préparation d'un additif tel que défini selon l'une des revendications 1 à 6, **caractérisé en ce qu'**il comprend une étape de réaction d'addition entre un acide gras R₁CO₂H et le caprolactame avec un rapport molaire dudit caprolactame par rapport audit acide gras allant de 0,8 à 3, de préférence de 0,9 à 2,75 et plus préférentiellement de 0,9 à 2,5, ledit procédé ne comprenant aucune étape de séparation ni de purification.

8. Composition aqueuse, en particulier de liant, **caractérisée en ce qu'**elle comprend en tant qu'hydrogélateur, au moins un additif tel que défini selon l'une des revendications 1 à 6 ou tel qu'obtenu par le procédé tel que défini selon la revendication 7.

9. Composition selon la revendication 8, **caractérisée en ce qu'**elle a un pH basique, de préférence d'au moins 9, plus préférentiellement d'au moins 10.

10. Composition selon la revendication 8 ou 9, **caractérisée en ce qu'**il s'agit d'une composition aqueuse de liant, de préférence une composition aqueuse de revêtements, en particulier de vernis, de peintures, d'encres ou une composition de colles ou d'adhésifs ou de cosmétique ou une composition de traitement de fibres ou de textiles avec ledit additif hydrogélateur étant utilisé comme un additif de rhéologie.

11. Composition selon la revendication 8 ou 9, **caractérisée en ce qu'**il s'agit d'une composition de tensioactif, en particulier de détergent ou d'agent décapant ou d'agent dépolluant ou d'agent floculant.

12. Utilisation d'un additif tel que défini dans l'une des revendications 1 à 6, en tant qu'additif hydrogélateur dans des compositions aqueuses.

13. Utilisation selon la revendication 12, **caractérisée en ce que** ledit additif est utilisé dans des compositions aqueuses de liant, plus particulièrement dans des compositions aqueuses de revêtements ou de colles ou d'adhésifs ou de cosmétique de traitement de fibres ou de traitement de textiles, en particulier comme additif de rhéologie.

14. Utilisation selon la revendication 12, **caractérisée en ce que** ledit additif est utilisé dans une composition de tensioactif, en particulier de détergent ou d'agent décapant ou d'agent dépolluant ou d'agent floculant.

15. Hydrogel, **caractérisé en ce qu'**il est le produit résultant de l'utilisation telle que définie selon l'une des revendications 12 à 14 d'un additif tel que défini selon l'une des revendications 1 à 6 ou obtenu par un procédé tel que défini selon la revendication 7.

16. Produit fini résultant de l'utilisation en tant qu'hydrogélateur, d'au moins un additif tel que défini selon l'une des revendications 1 à 6 ou obtenu par le procédé tel que défi ni selon la revendication 7, en particulier sélectionné parmi : revêtement ou colle ou adhésif ou cosmétique ou fibre traitée ou textile traité ou détergent ou agent décapant ou agent dépolluant ou agent floculant.

## Patentansprüche

1. Hydrogelierungadditiv auf Fettamid-Basis, **dadurch gekennzeichnet, dass** es das Produkt einer Additionsreaktion einer Fettsäure R₁CO₂H an Caprolactam ist und eine terminale Carbonsäurefunktion in Säureform oder in Salzform trägt durch ein Neutralisierungsmittel und dass das Additiv aus einer Mischung aus 3 unterschiedlichen Amidverbindungen besteht oder diese umfasst, die aus der Reaktion resultieren und durch die Zahl von n eingearbeiteten Caprolactam-Einheiten gekennzeichnet sind, wobei diese Zahl 1, 2 bzw. 3 beträgt, wobei eine mittlere Zahl n von Einheiten (Durchschnitt pro Molekül) von 0,8 bis 3, vorzugsweise von 0,9 bis 2,75 und stärker bevorzugt von 0,9 bis 2,5 reicht.

2. Additiv nach Anspruch 1, **dadurch gekennzeichnet, dass** die 3 Verbindungen durch die folgende Formel (I) definiert sind und n = 1, n = 2 bzw. n = 3 entsprechen
R₁-C(=O)-[NH(CH₂)₅C(=O)-]ₙ₋₁-NH(CH₂)₅-CO₂M (I)
wobei im Falle der Salzform M -H oder ein Kation ist, wobei das Kation mit dem Neutralisierungsmittel verbunden ist.

3. Additiv nach Anspruch 1 oder 2, **dadurch gekennzeichnet, dass** das Fettamin eine Anzahl an Kohlenstoffatomen umfasst, die von 10 bis 24 reicht.

4. Additiv nach einem der Ansprüche 1 bis 3, **dadurch gekennzeichnet, dass** das Additiv in Form eines mikronisierten Pulvers vorliegt, das vorzugsweise eine volumenmittlere Größe unter 50 µ, vorzugsweise unter 25 µ aufweist.

5. Additiv nach einem der Ansprüche 1 bis 4, **dadurch gekennzeichnet, dass** es in Salzform vorliegt und dass das Neutralisierungsmittel aus organischen oder mineralischen Basen ausgewählt ist.

6. Additiv nach einem der Ansprüche 1 bis 5, **dadurch gekennzeichnet, dass** es in Form eines Gels in Wasser oder als Hydrogelbildner vorliegt, stärker bevorzugt mit einem Gewichtsanteil von weniger als 5 % und noch stärker bevorzugt mit einem Anteil, der 1 Gew.-% nicht übersteigt.

7. Verfahren zur Herstellung eines Additivs, wie es in einem der Ansprüche 1 bis 6 definiert ist, **dadurch gekennzeichnet, dass** es einen Schritt einer Additionsreaktion zwischen einer Fettsäure R₁CO₂H und dem Caprolactam umfasst, wobei das Molverhältnis des Caprolactams zu der Fettsäure von 0,8 bis 3, vorzugsweise von 0,9 bis 2,75 und stärker bevorzugt von 0,9 bis 2,5 reicht, wobei das Verfahren keinen Schritt der Trennung oder Reinigung umfasst.

8. Wässrige Zusammensetzung, insbesondere Bindemittelzusammensetzung, **dadurch gekennzeichnet, dass** sie als Hydrogelbildner wenigstens ein Additiv umfasst, wie es in einem der Ansprüche 1 bis 6 definiert ist oder wie es durch das Verfahren erhalten wird, wie es in Anspruch 7 definiert ist.

9. Zusammensetzung nach Anspruch 8, **dadurch gekennzeichnet, dass** sie einen basischen pH, vorzugsweise mindestens 9, stärker bevorzugt mindestens 10, aufweist.

10. Zusammensetzung nach Anspruch 8 oder 9, **dadurch gekennzeichnet, dass** es sich um eine wässrige Bindemittelzusammensetzung, vorzugsweise eine wässrige Beschichtungszusammensetzung, insbesondere eine Lack-, Anstrich- oder Tintenzusammensetzung oder eine Klebstoff- oder Haftmittelzusammensetzung oder Kosmetika-zusammensetzung oder eine Zusammensetzung zur Behandlung von Fasern oder Textilien handelt, wobei das Hydrogelierungadditiv als Rheologieadditiv verwendet wird.

11. Zusammensetzung nach Anspruch 8 oder 9, **dadurch gekennzeichnet, dass** es sich um eine Tensidzusammensetzung handelt, insbesondere eine Detergens- oder Beizmittel- oder Schadstoffentfernungsmittel- oder Flockungsmittelzusammensetzung.

12. Verwendung eines Additivs, wie es in den Ansprüchen 1 bis 6 definiert ist, als Hydrogelierungadditiv in wässrigen Zusammensetzungen.

13. Verwendung nach Anspruch 12, **dadurch gekennzeichnet, dass** das Additiv in wässrigen Bindemittelzusammensetzungen, spezieller in wässrigen Beschichtungs- oder Klebstoff- oder Haftmittel- oder Kosmetika-Faserbehandlungs- oder Textilbehandlungszusammensetzungen, verwendet wird, insbesondere als Rheologieadditiv.

14. Verwendung nach Anspruch 12, **dadurch gekennzeichnet, dass** das Additiv in einer Tensidzusammensetzung, insbesondere einer Detergens- oder Beizmittel- oder Schadstoffentfernungsmittel- oder Flockungsmittelzusammensetzung verwendet wird.

15. Hydrogel, **dadurch gekennzeichnet, dass** es das Produkt ist, das aus der wie in einem der Ansprüche 12 bis 14 definierten Verwendung eines Additivs resultiert, wie es in einem der Ansprüche 1 bis 6 definiert ist, oder das durch ein Verfahren erhalten wird, wie es in Anspruch 7 definiert ist.

16. Endprodukt, resultierend aus der Verwendung mindestens eines Additivs, wie es in einem der Ansprüche 1 bis 6 definiert ist oder das durch ein Verfahren erhalten wird, wie es in Anspruch 7 definiert ist, als Hydrogelbildner, wobei es insbesondere aus einer Beschichtung oder einem Klebstoff oder einem Haftmittel oder einem Kosmetikum oder einer behandelten Faser oder einem behandelten Textil oder einem Detergens oder einem Beizmittel oder einem Schadstoffentfernungsmittel oder einem Flockungsmittel ausgewählt ist.

## Claims

1. Hydrogelating additive based on fatty amide, **characterized in that** it is the product of the addition reaction of a fatty acid R₁CO₂H with caprolactam and carries an end carboxyl functional group in the acid form or in the form salified by a neutralizing agent and **in that** the said additive consists of or comprises the mixture of 3 different amide compounds resulting from the said reaction and which are **characterized by** the number n of caprolactam units incorporated, which number is respectively 1, 2 and 3, with a mean number n of units (mean per molecule) ranging from 0.8 to 3, preferably from 0.9 to 2.75 and more preferably from 0.9 to 2.5.

2. Additive according to Claim 1, **characterized in that** the said 3 compounds are defined according to the following formula (I) and respectively correspond to n = 1, n = 2 and n = 3
R₁-C(=O)-[NH(CH₂)₅C(=O)-]ₙ₋₁-NH(CH₂)₅-CO₂M (I)
with M being -H or a cation in the case of the said salified form, the said cation being related to the said neutralizing agent.

3. Additive according to Claim 1 or 2, **characterized in that** the said fatty acid comprises a number of carbon atoms ranging from 10 to 24.

4. Additive according to one of Claims 1 to 3, **characterized in that** the said additive is in the form of a micronized power, preferably having a volume-average size of less than 50 µ, preferably less than 25 µ.

5. Additive according to one of Claims 1 to 4, **characterized in that** it is in the salified form and **in that** the said neutralizing agent is selected from organic or inorganic bases.

6. Additive according to one of Claims 1 to 5, **characterized in that** it is in the gel form in water or as hydrogelator and more preferably at a content by weight of less than 5% and more preferably still at a content not exceeding 1% by weight.

7. Process for the preparation of an additive as defined according to one of Claims 1 to 6, **characterized in that** it comprises a stage of an addition reaction between a fatty acid R₁CO₂H and caprolactam with a molar ratio of the said caprolactam with respect to the said fatty acid ranging from 0.8 to 3, preferably from 0.9 to 2.75 and more preferably from 0.9 to 2.5, the said process not comprising any separation or purification stage.

8. Aqueous composition, in particular aqueous binder composition, **characterized in that** it comprises, as hydrogelator, at least one additive as defined according to one of Claims 1 to 6 or as obtained by the process as defined according to Claim 7.

9. Composition according to Claim 8, **characterized in that** it has a basic pH, preferably a pH of at least 9, more preferably of at least 10.

10. Composition according to Claim 8 or 9, **characterized in that** it is an aqueous binder composition, preferably an aqueous coating composition, in particular an aqueous varnish, paint or ink composition, or an adhesive composition or a cosmetic composition or a composition for the treatment of fibres or textiles, with the said hydrogelating additive being used as a rheology additive.

11. Composition according to Claim 8 or 9, **characterized in that** it is a surfactant composition, in particular a detergent or stripping agent or depolluting agent or flocculating agent composition.

12. Use of an additive as defined in one of Claims 1 to 6 as hydrogelating additive in aqueous compositions.

13. Use according to Claim 12, **characterized in that** the said additive is used in aqueous binder compositions, more particularly in aqueous coating or adhesive or cosmetic compositions for the treatment of fibres or for the treatment of textiles, in particular as rheology additive.

14. Use according to Claim 12, **characterized in that** the said additive is used in a surfactant composition, in particular a detergent or stripping agent or depolluting agent or flocculating agent composition.

15. Hydrogel, **characterized in that** it is the product resulting from the use as defined according to one of Claims 12 to 14 of an additive as defined according to one of Claims 1 to 6 or obtained by a process as defined according to Claim 7.

16. Finished product resulting from the use as hydrogelator of at least one additive as defined according to one of Claims 1 to 6 or obtained by the process as defined according to Claim 7, in particular selected from: coating or adhesive or cosmetic or treated fibre or treated textile or detergent or stripping agent or depolluting agent or flocculating agent.
